# EUROPEAN PATENT SPECIFICATION

(11) **EP 3 013 244 B1**
(45) Date of publication and mention of the grant of the patent: **16.01.2019**
(21) Application number: 14744162.0
(22) Date of filing: 16.06.2014
(51) Int. Cl.: A61B 8/08, G01S 7/52

(54) **SYSTEM AND METHOD FOR MAPPING ULTRASOUND SHEAR WAVE ELASTOGRAPHY MEASUREMENTS**
SYSTEM UND VERFAHREN ZUR ABBILDUNG VON ULTRASCHALLSCHERWELLENELASTOGRAFIEMESSUNGEN
SYSTÈME ET PROCÉDÉ DE MISE EN CORRESPONDANCE DE MESURES D'ÉLASTOGRAPHIE ULTRASONORE PAR ONDES DE CISAILLEMENT

(30) Priority: 26.06.2013 US 201361839668 P
(43) Date of publication of application: 04.05.2016
(73) Proprietor: Koninklijke Philips N.V., 5656 AE Eindhoven (NL)
(72) Inventor: XIE, Hua, 5656 AE Eindhoven (NL); PARTHASARATHY, Vijay, 5656 AE Eindhoven (NL); ZHOU, Shiwei, 5656AE Eindhoven (NL); ROBERT, Jean-Luc, 5656 AE Eindhoven (NL); SHAMDASANI, Vijay Thakur, 5656 AE Eindhoven (NL)
(74) Representative: Gravendeel, Cornelis
(86) International application number: PCT/IB2014/062257
(87) International publication number: WO 2014/207605

(56) References cited:
- EP-A1- 2 476 376
- WO-A1-2013/050899
- US-A1- 2012 133 663
- US-A1- 2013 116 542

## Description

### FIELD OF THE INVENTION

The present invention relates to an ultrasound elastography system for providing an elastography measurement result of an anatomical site and an ultrasound elastography method for inspecting an anatomical site with an ultrasound elastography system. The present invention further relates to a computer program for implementing such method.

### BACKGROUND OF THE INVENTION

Liver fibrosis and cirrhosis are a response to chronic liver injury from a variety of causes including viral, autoimmune, drug induced, cholestatic and metabolic diseases. Liver cirrhosis may ultimately lead to hepatic failure and is associated with primary liver cancer hepatocellular carcinoma, which increases the relative mortality rate.

Staging and quantifying the extent of liver fibrosis is important in the management of patients with chronic liver disease. Liver biopsy is considered the "gold standard" for diagnosis and staging of liver fibrosis. However, liver biopsy can cause discomfort and anxiety to patients. Significant complications occur in 1-5% of patients with a reported mortality rate of between 1:1000 and 1:10,000. Liver biopsy samples contain only about 1/50,000 of the liver and so are limited by sampling variation and further observer variability during histological assessment.

Limitations of liver biopsy have led to the development of various non-invasive evaluations of liver fibrosis that are more suitable for screening, treatment monitoring, and follow-up. Ultrasound shear wave elastography technique emerges as a technique for non-invasive liver fibrosis staging due to its absolute stiffness quantification capability, real-time, cost-efficient and portable features. Commercial products are already available, for example the Philips Ultrasound new release shear wave Elastography Point Quantification (ElastPQ). With such a method, acoustic radiation force is used to stress the liver or any other anatomical site mechanically and produce a shear wave. The resulting tissue displacement is measured and used to estimate the elasticity of the anatomical site, which has been found to correlate with fibrosis stage in the case of a liver being the anatomical site.

Due to system limitations such as transducer heating and physical limitations such as shear wave fast attenuation, current commercial shear wave elastography products usually only provide measurement at a user selected point location or a bigger region of interest (ROI) spatially confined within the B-mode field of view. In a typical workflow, the user selects a suspicious region under the conventional ultrasound B-mode imaging, activates shear wave elastography tool, makes measurements, and repeats the process at another user selected position. A measurement made in the ROI in the imaging plane may then be displayed as a value associated with the ROI, for example on a display of the ultrasound elastography system. For example, by this, a liver stiffness value associated with the ROI may be reported in the unit of Young's modulus (kPa) or shear wave speed (m/s). By moving the ROI, a user may inspect a liver in a non-invasive manner.

It is recommended to make multiple stiffness measurements, for example in the order of ten, distributed in the entire liver to have a comprehensive evaluation of fibrosis level. This is cumbersome and time consuming. Further, it leaves the examiner in doubt about the exact distribution or pattern of the measurement results.

Document WO 2012/078280 A1 shows a system for generating elastography images of tissue includes a scanning device configured to be moved over the tissue. The scanning device includes an ultrasound transducer and a shear wave transducer, wherein the shear wave transducer is configured to direct shear waves into the tissue during operation of the ultrasound transducer. A location and orientation sensor system is coupled with the scanning device, a receiver configured to receive images from the ultrasound transducer, a receiver configured to receive location and orientation data from the sensor system for each image, and a viewer configured to provide a rapid, sequential display of the received images.

There is a need to further improve such elastography system.

US2012133663A1 discloses an ultrasound elastography system and method according to the preambles of claims 1 and 10.

### SUMMARY OF THE INVENTION

It is an object of the present invention to provide an improved ultrasound elastography system and method. It is a further object of the present invention to provide a computer program for implementing such method.

In a first aspect of the present invention anultrasound elastography system for providing a shear wave elastography measurement result of an anatomical site according to claim 1 is presented.

In a further aspect of the present invention, an ultrasound elastography method for inspecting an anatomical site with an ultrasound elastography system according to claim 10 is presented.

In a third aspect of the present invention a, preferably non-transitory, computer program comprising program code means for causing a computer to carry out the steps of the method according to the second aspect or one of tis refinements when said computer program is carried out on a computer.

The basic idea of the invention is to present an integrated system and method to localize elastography measurement positions and a biopsy position, in particular in a liver three-dimensional anatomy. Shear wave ultrasound elastography imaging and localization can be realized using two approaches, namely position sensing of the transducer or pure image based registration. In the first approach, a sensor will be placed on the transducer for real-time monitoring of its positions and orientation using electromagnetic or optical tracking systems. In the second approach, a subset of ultrasound volume or cross-plane images where stiffness measurements are made are registered to extensive three-dimensional liver anatomy. The main elements of an ultrasound system may comprise a one-dimensional or two-dimensional transducer that is capable of conventional B-mode imaging and shear wave elastography, a three-dimensional liver anatomy image acquired by sweeping a one-dimensional ultrasound transducer, or a two-dimensional ultrasound transducer, or a previously acquired three-dimensional image acquires by other modalities such as CT/MRI, an electromagnetic or optical tracking system, and an application that comprises a data acquisition system that communicates with the ultrasound system to get stiffness measurement relative positions and ultrasound images, a data acquisition system that communicates with the tracking system mapping stiffness measurement and biopsy locations using tracking system or mapping stiffness measurement locations using image registration approach, and a display solution for three-dimensional rendering and visualizing stiffness measurement locations, a quantification tool that can calculate the distance between any stiffness measurement location and the biopsy location.

The combination of shear wave elastography and tracking could have broad application in diagnostic imaging and interventional oncology procedure and can be included in future ultrasound imaging systems. Besides liver fibrosis staging, wherein the anatomical site is a liver, the system and technique could have applications in visualizing and mapping stiffness measurement and biopsy location in staging breast, thyroid and prostate cancer. Hence, the anatomical site may also be a breast, a thyroid or a prostate.

Preferred embodiments of the invention are defined in the dependent claims. It shall be understood that the claimed method has similar and/or identical preferred embodiments as the claimed device and as defined in the dependent claims.

According to a refinement of the system, the system further comprises a tracking device to track a position and an orientation of the portable ultrasound image acquisition probe. By this, it is possible to track a position and orientation of the probe and to use this information to determine the location of a shear wave elastography measurement result.

In a further refinement of the system, the ultrasound signal and image processing assembly is further configured to determine the location of the shear wave elastography measurement result within the three-dimensional image of the anatomical site by tracking a position and an orientation of portable ultrasound image acquisition probe via the tracking device. By this, the tracking device may be used to map the position of elastography measurements and to visualize them in the three-dimensional image to the user.

In a further refinement of the system, the system further comprises a biopsy device, and wherein the ultrasound signal and image processing assembly is further configured to tracking a location of a biopsy device within the three-dimensional image of the anatomical site via the tracking device. By this, it is further possible to track a position of the biopsy device. By this, a guidance of the biopsy workflow via the system can be provided. Further, positions of a biopsy sample can be compared to elastography measurement locations or it can be ensured that a site of a biopsy sample is congruent with a prior determined elastographic measurement result.

In a further refinement of the system, the ultrasound signal imaging processing assembly is further configured to determine a distance between a location of a shear wave elastography measurement result and the location of a biopsy device. By this, it can be determined whether a side of a biopsy sample is in the proximity of a region of interest in which an elastography measurement has been conducted.

The system comprises a user input device enabling a user to select a region of interest within the ultrasound image in which region of interest an elastography measurement is to be conducted. By this, it is possible for a user to select the region of interest in the ultrasound image, in particular, a commonly known B-mode ultrasound image so that the elastography measurement is conducted in a region identified by the user.

In a further refinement of the system, the ultrasound signal and image processing assembly is further configured to determine the location of the shear wave elastography measurement result within the three-dimensional image of the anatomical site by registering the ultrasound image in which the region of interest is selected with the three-dimensional image of the anatomical site. By this, the registration can be conducted only by the ultrasound signal image processing assembly in a computer-implemented manner. Hence, further equipment, for example the tracking device, can be avoided to properly align the biopsy measurement results with the three-dimensional image and the elastographic measurement locations.

In a further refinement of the system, the ultrasound image is a two-dimensional ultrasound image. In particular, the ultrasound image can be a common known bi-mode ultrasound image. By this, conventional ultrasound imaging techniques can be used to provide the user with a two-dimensional view in order to identify locations for elastographic measurement and to guide a biopsy process, for example.

In a further refinement of the system, the three-dimensional image of the anatomical site is a three-dimensional image acquired via a modality different from ultrasound image acquisition, wherein the three-dimensional image of the anatomical site is stored in the memory unit of the ultrasound signal and image processing assembly. By this, the three-dimensional images of the whole anatomical site, for example a liver, can be used, in particular the different modality may be computer-tomographic imaging or magnetic resonance imaging.

In a further refinement of the system, the portable ultrasound image acquisition probe and the ultrasound signal and image processing assembly are further configured to enable a user to acquire the three-dimensional ultrasound image of the anatomical site and to store it as the three-dimensional image of the anatomical site. By this, the user can be enabled to acquire the three-dimensional ultrasound image prior to the elastographic measurement examination of the biopsy examination via the same system.

The method further comprises the step of selecting a region of interest within an ultrasound image acquired with the portable ultrasound image acquisition probe in which region of interest an elastography measurement is to be conducted, and wherein the step of determining a location of a shear wave elastography measurement result within the three-dimensional image of the anatomical site is conducted by registering the ultrasound image with the three-dimensional image of the anatomical site. By this, comparable advantages can take place in the method. Registration of the locations of elastographic measurement results with the three-dimensional image can be conducted purely in a computer-implemented manner without the need of any additional equipment.

In a refinement of the method, the method further comprises the step of tracking a position and an orientation of portable ultrasound image acquisition probe via a tracking device. By this, a tracking device may be used to track the position and orientation of the portable ultrasound image acquisition device. For example, an electromagnetic scanning device or an optical scanning device may be used. By this, the mapping of positions of the elastographic measurement with, for example, biopsy locations and/or the three-dimensional image of the anatomical site, for example the liver, can be supported.

In a further refinement of the method, the method further comprises the steps of tracking a location of a biopsy device within the three-dimensional image of the anatomical site via the tracking device, and determining a distance between a location of a shear wave elastography measurement result and the location of a biopsy device.

By this, the location of biopsy samples can be validated within the three-dimensional image and the relation to the elastographic measurement results can be determined.

### BRIEF DESCRIPTION OF THE DRAWINGS

These and other aspects of the invention will be apparent from and elucidated with reference to the embodiment(s) described hereinafter. In the following drawings
Fig. 1 shows an in vivo liver stiffness measurement in three imaging planes of a liver to illustrate the background of the current invention.
Fig. 2 shows a schematic illustration of a further embodiment of an ultrasound elastography system,
Fig. 3 shows a block diagram illustrating the different components of an exemplary ultrasound elastography system,
Fig. 4 shows a schematic illustration showing planes and angles in ultrasound volume scanning,
Fig. 5 shows an example of a B-mode image with an ROI for an elastography measurement,
Fig. 6 shows an illustration of an embodiment including a tracking device related to a patient,
Fig. 7 shows an embodiment of a method,
Fig. 8 shows a first use example of a mapped elastography measurement,
Fig. 9 shows a second use example of a mapped elastography measurement, and
Fig. 10 shows a third use example of a mapped elastography measurement.

### DETAILED DESCRIPTION OF THE INVENTION

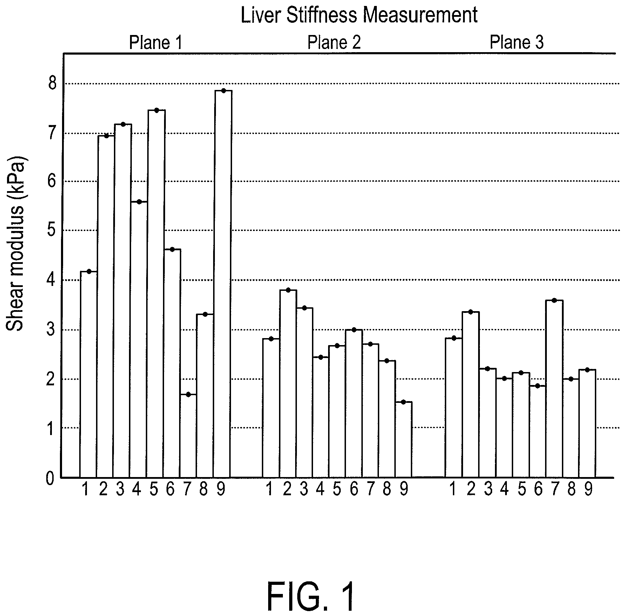

Fig. 1 shows an in vivo liver stiffness measurement in three imaging planes of a liver for one chronic liver patient. In each plane, nine measurements are made.

Although liver fibrosis is considered a diffuse parenchymal disease, fibrosis actually varies in severity and location throughout the liver. Clinical findings suggest that there is natural variation in amount of fibrosis (stage) and location of fibrosis. For example, as shown in Fig. 1, liver stiffness is measured for a liver patient in three imaging planes of the right liver lobe by Philips ElastPQ feature. In each imaging plane, nine measurements are made at nine different locations. It is clear that on average liver is stiffer in plane 1 than in plane 2 and 3, indicating inhomogeneous fibrosis distribution in viral hepatitis. Using a stiffness cut-off value derived from the clinical study, it may lead to average appraisal of fibrosis stage FO-F1 (no-mild fibrosis) for this patient using the Metavir scoring system. However a diagnosis in a more localized way may be conducted, we have F0-F1 (no fibrosis) for imaging planes 2 and 3 but F ≥2 (stage 2 fibrosis or higher, significant fibrosis) in imaging plane 1. As a result, different diagnosis results and clinical decision making may be applied.

The above clinical example illustrates again why liver biopsy is subject to sampling error: a liver biopsy usually examines just one area of the liver for scar tissue and damaged liver cells. To help doctors better diagnose the severity and location of fibrosis throughout the liver, there is a need to map the shear wave measurement locations in the entire liver, correlate with the biopsy location, and visualize these locations in three-dimensional liver volume. Such mapping capabilities can, as outlined in detail below, localize the stiffness measurement positions with respect to the liver anatomy, establish spatial relationship between stiffness measurements and liver fibrosis for accurate fibrosis staging, and enable longitudinal monitoring anti-fibrotic therapy treatment.

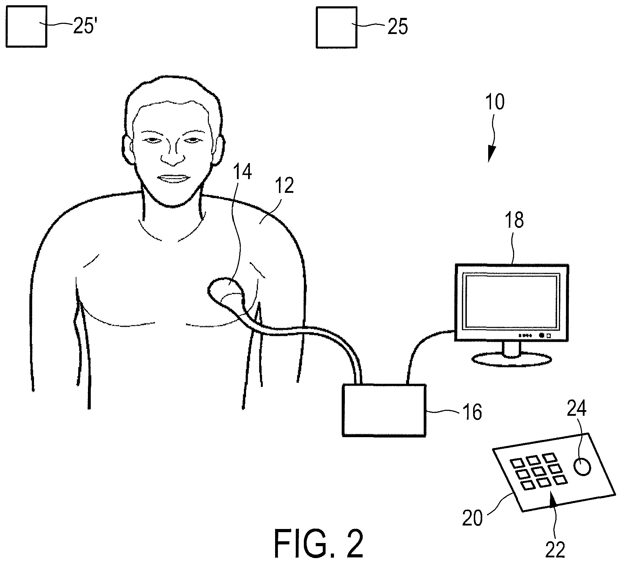

Fig. 2 shows a schematic illustration of an ultrasound elastography system 10 according to a further embodiment, in particular a medical ultrasound three-dimensional imaging system. The ultrasound elastography system 10 is applied to inspect an anatomical site of an anatomical site, in particular an anatomical site of a patient 12. The ultrasound elastography system 10 comprises an ultrasound image acquisition probe 14 having at least one transducer array having a multitude of transducer elements for transmitting and/or receiving ultrasound waves. In one example, the transducer elements each can transmit ultrasound waves in form of at least one transmit impulse of a specific pulse duration, in particular a plurality of subsequent transmit pulses. The transducer elements can for example be arranged in a one-dimensional row, for example for providing a two-dimensional image that can be moved or swiveled around an axis mechanically. Further, the transducer elements may be arranged in a two-dimensional array, in particular for providing a multi-planar or three-dimensional image.

In general, the multitude of two-dimensional images, each along a specific acoustic line or scanning line, in particular scanning receive line, may be obtained in three different ways. First, the user might achieve the multitude of images via manual scanning. In this case, the ultrasound image acquisition probe may comprise position-sensing devices that can keep track of a location and orientation of the scan lines or scan planes. However, this is currently not contemplated. Second, the transducer may be automatically mechanically scanned within the ultrasound image acquisition probe. This may be the case if a one dimensional transducer array is used. Third, and preferably, a phased two-dimensional array of transducers is located within the ultrasound image acquisition probe and the ultrasound beams are electronically scanned. The ultrasound image acquisition probe may be hand-held by the user of the system, for example medical staff or a doctor. The ultrasound image acquisition probe 14 is applied to the body of the patient 12 so that an image of an anatomical site 32 in the patient 12 is provided.

Further, the ultrasound elastography system 10 has an ultrasound signal and image processing assembly 16 that controls the provision of an ultrasound image via the ultrasound elastography system 10. The ultrasound signal and image processing assembly 16 controls not only the acquisition of data via the transducer array of the ultrasound image acquisition probe 14 but also signal and image processing that form the ultrasound images out of the echoes of the ultrasound beams received by the transducer array of the ultrasound image acquisition probe 14. Further, the ultrasound signal and image processing assembly 16 determines stiffness values under the elastography mode, in particular out of the echoes of the ultrasound beams received by the transducer array of the ultrasound image acquisition probe 14.

The ultrasound elastography system 10 further comprises a display 18 for displaying the three-dimensional images to the user. Further, an input device 20 is provided that may comprise keys or a keyboard 22 and further input devices, for example a track ball 24. The input device 20 might be connected to the display 18 or directly to the ultrasound signal and image processing assembly 16.

Further, the ultrasound elastography system 10 comprises a tracking device, for example an electromagnetic tracking device. Parts of the tracking device are situated within the probe 14 or may be associated with the probe via a clip. Further parts 25, 25', for example sensors like magnetoresistive sensors or optical sensors, may be placed in the circumference of the ultrasound elastography system. Preferably, the spatial coordinates of the further parts 25, 25' are known.

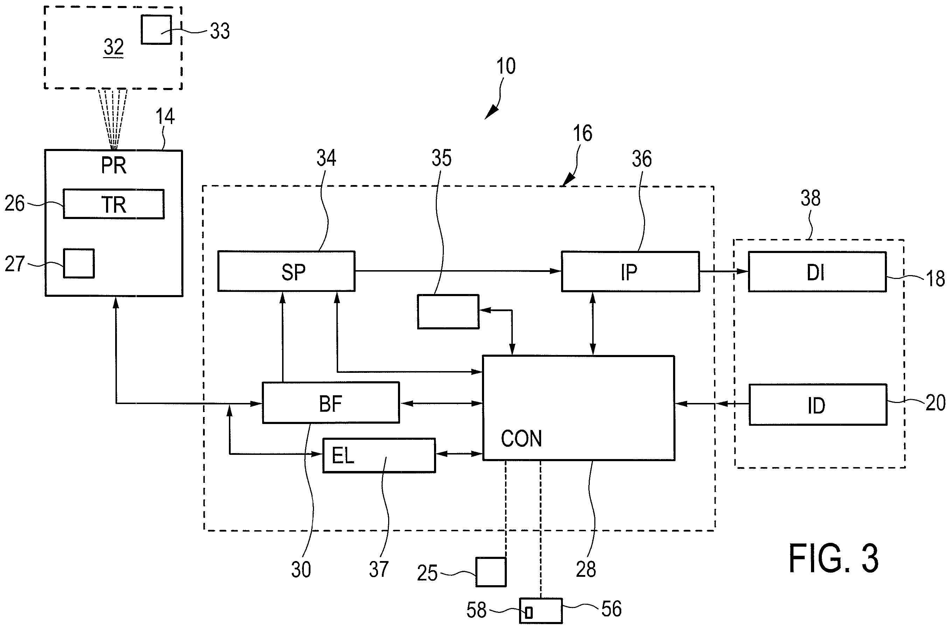

Fig. 3 shows a schematic block diagram of the ultrasound elastography system 10. As already laid out above, the ultrasound elastography system 10 comprises an ultrasound image acquisition probe (PR) 14, the ultrasound signal and image processing assembly (CU) 16, the display (DI) 18 and the input device (ID) 20. As further laid out above, the probe (PR) 14 comprises a transducer array 26, for example a phased two-dimensional transducer array or automatically scanned one-dimensional transducer array. Further, the probe comprises a part 27 of the tracking device, for example a coil that generates an electromagnetic field that is sensed via the sensors 25, 25' or a fiducial, in particular to support optical tracking. In general, the ultrasound signal and image processing assembly (CU) 16 may comprise a central processing unit that may include analog and/or digital electronic circuits, a processor, microprocessor or the like to coordinate the whole image acquisition and provision. Further, the ultrasound signal and image processing assembly 16 comprises a herein called central processing unit 28. However, it has to be understood that the central processing unit 28 does not need to be a separate entity or unit within the ultrasound elastography system 10. A memory unit is indicated by reference numeral 35. It can be a part of the ultrasound signal and image processing assembly 16 and generally be hardware or software implemented. The current distinction is made for illustrative purposes only. The central processing unit (CON) 28 as part of the ultrasound signal and image processing assembly 16 may control a beam former and, by this, what images of the anatomical site 32 are taken and how these images are taken. The beam former (BF) 30 generates the voltages that drives the transducer array (TR) 26, determines parts repetition frequencies, it may scan, focus and apodize the transmitted beam and the reception or receive beam(s) and may further amplify filter and digitize the echo voltage stream returned by the transducer array 26. Further, the central processing unit 28 of the ultrasound signal and image processing assembly 16 may determine general scanning strategies. Such general strategies may include a desired anatomical site acquisition rate, lateral extent of the anatomical site, an elevation extent of the anatomical site, maximum and minimum line densities, scanning line times and the line density as already explained above. The beam former 30 further receives the ultrasound signals from the transducer array 26 and forwards them as image signals.

A shear wave elastography subsystem is designated with reference numeral 37. The shear wave subsystem 37 allows the ultrasound imaging system 10 to operate in a shear wave mode. In certain embodiments, the shear wave subsystem 37 determines the focus locations and sequence for moving a shear wave source among the focus locations. In addition, or in other embodiments, the shear wave subsystem 37 performs the other functions described herein with respect to dynamically controlling the shear wave front. An artisan will recognize from the disclosure herein that the shear wave subsystem 37 may be combined with other components of the system 10. For example, at least some of the functions described for the shear wave subsystem 37 may be performed by the CON 28. For tissue quantification according to one embodiment, the shear wave subsystem 37 may allow the user of the ultrasound imaging system 10 to identify an anatomic location for measurement using a region of interest marker placed on an ultrasound image. An acoustic push pulse is applied just lateral to this location, inducing a shear wave that travels through the region of interest. Tracking beams, sensitive to greater than 1/100 the wavelength of sound, are applied to the shear wave propagation path. The tracking beams are continuously transmitted until the passing shear wave front is detected. The time between generation of the shear wave and detection of the peak is utilized to compute the shear wave velocity. Multiple measurements are made for a given spatial location before a value is reported in order to ensure measurement quality.

Further, the ultrasound elastography system 10 comprises a signal processor (SP) 34 that receives the image signals. The signal processor 34 is generally provided for analogue-to-digital-converting, digital filtering, for example, band pass filtering, as well as the detection and compression, for example a dynamic range reduction, of the received ultrasound echoes or image signals. The signal processor forwards image data.

Further, the ultrasound elastography system 10 comprises an image processor (IP) 36 that converts image data received from the signal processor 34 into display data finally shown on the display 18. In particular, the image processor 36 receives the image data, preprocesses the image data and may store it in an image memory. These image data is then further post-processed to provide images most convenient to the user via the display 18. In the current case, in particular, the image processor 36 may form the three-dimensional images out of a multitude of two-dimensional images in each slice.

A user interface is generally depicted with reference numeral 38 and comprises the display 18 and the input device 20. It may also comprise further input devices, for example, a mouse or further buttons which may even be provided on the ultrasound image acquisition probe 14 itself.

Further, reference numeral 56 designates a biopsy device that may be part of the ultrasound system 10. Similar to the probe 14, the biopsy device 56 may comprise a part 58 of the tracking device 25, for example a coil that generates an electromagnetic field that is sensed via the sensors 25, 25' or a fiducial, in particular to support optical tracking.

A particular example for an ultrasound elastography system which may apply the current invention is a system applying the Philips ultrasound new release shear wave Elastography Point Quantification (ElastPQ).

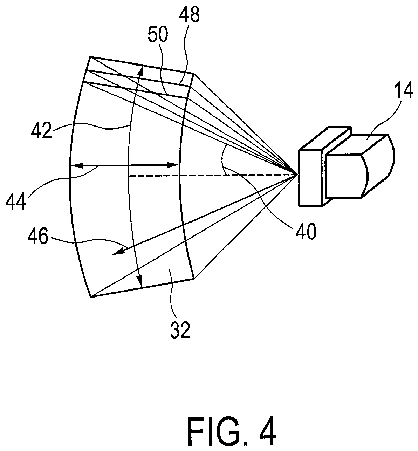

Fig. 4 shows an example of an anatomical site 32 relative to the ultrasound image acquisition probe 14. The exemplary anatomical site 32 depicted in this example is of a sector type, due to the transducer array of the ultrasound image acquisition probe 14 being arranged as a phased two-dimensional electronically scanned array. Hence, the size of the anatomical site 32 may be expressed by an elevation angle 42 and a lateral angle 44. A depth 46 of the anatomical site 32 may be expressed by a so-called line time in seconds per line. That is the scanning time spent to scan a specific scanning line.

The anatomical site 32 may be divided into a multitude of slices 48, 50 or two-dimensional images. Only two slice 48, 50 are depicted for illustrative purposes. Actually, a multitude of planes or slices 48, 50 having different elevational angles 40 are spread over the volume of the anatomical site 32. Of course, the slices 48, 50 may also be oriented in the elevational direction and spread across the anatomical site 32 in the lateral direction. During image acquisition, the two-dimensional transducer array of the ultrasound image acquisition probe 14 is operated by a beam former in a way that the anatomical site 32 is scanned along a multitude of these scan lines within each of the slices 48, 50 sequentially. In multi-line receive processing, a single transmit beam might illuminate a multitude, for example four, receive scanning lines along which signals are acquired in parallel. If so, such sets of receive lines are then electronically scanned across the anatomical site 32 sequentially.

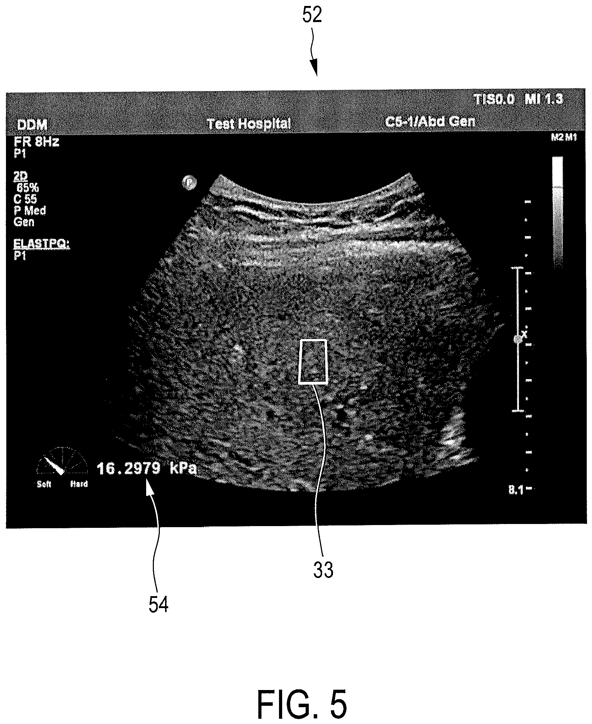

Fig. 5 shows an ultrasound image 52 that is a conventional B-mode ultrasound image. The ultrasound image is two-dimensional and could, for example, be acquired with the system 10 as explained above. Further, it is shown a region of interest 33 which a user may use to select a certain point within the B-mode image 52 for elastography measurement. After a user selects a certain region of interest 33 within the image 52, an elastography measurement is conducted and a result is displayed as value in the image 52 which value is generally designated by reference numeral 54. By this, the user may evaluate different portions of the B-mode image 52 to map the tissue elasticity over the B-mode image. While in Fig. 5 a proper placement of a region of interest 33 is shown, this may not always be the case.

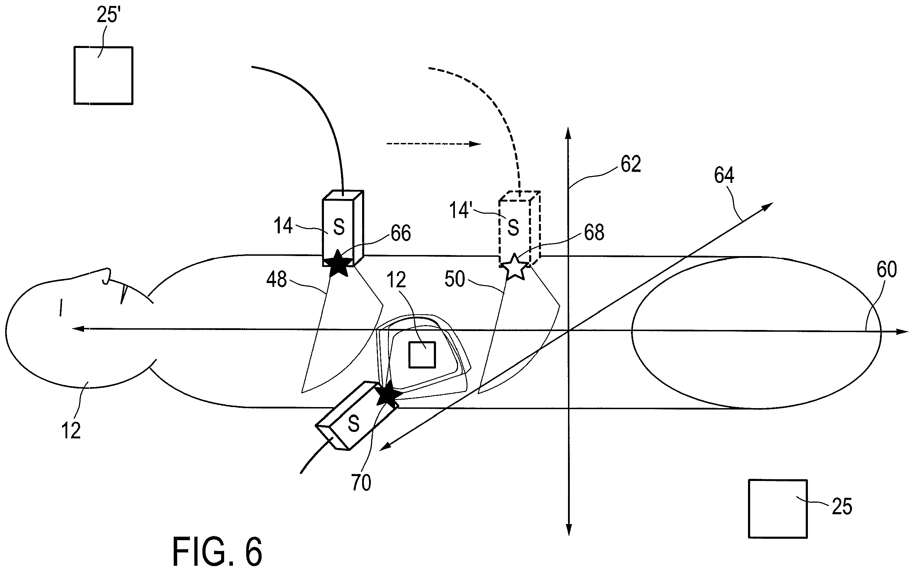

Fig. 6 shows a schematic illustration of the body of a patient 12. The cranio-caudal axis is designated with reference numeral 60. The anterior-posterior axis is designated with reference numeral 62. The left-right axis is designated with reference numeral 64. The user moves the probe to a first position 66 which is tracked by the tracking device 25, 25'. This may be conducted, for example, by an electromagnetic tracking device or an optical tracking device.

By this, it is possible to track the different positions of the probe 14. For example, if the probe 14 is moved from a first location 66 to a second location 68 or a third location 70, the corresponding position and orientation of the probe 14 can be tracked and, hence, the position and orientation of a plane 48, 50 in which the two-dimensional ultrasound imaging and, hence, the elastographic measurement, took place can be determined. By this, not only the position of the plane 48, 50 with reference to the body of the patient 12 can be determined but also the position and orientation of the plane 48, 50 in an absolute coordinate system. This enables any registration of the planes 48, 50 with previously acquired three-dimensional images of anatomical sites that have a known position and orientation as well.

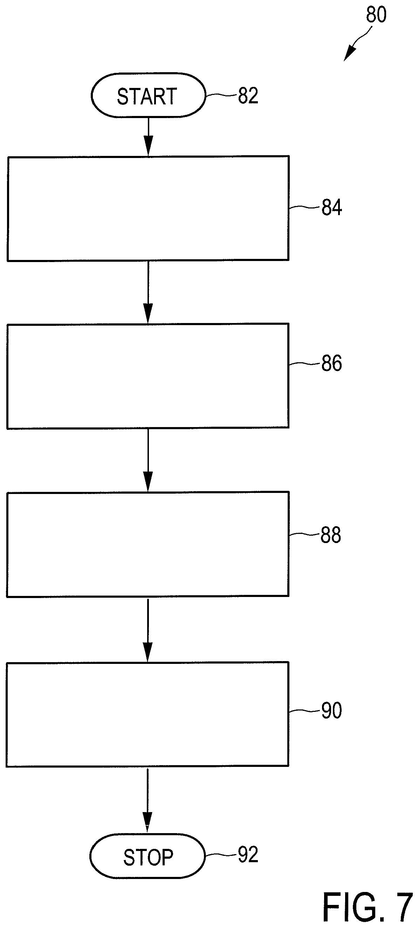

Fig. 7 shows an embodiment of a method 80. The method starts in a step 82. Then, in step 84, it is acquired a three-dimensional image of the anatomical site 32. This three-dimensional image may to be acquired via the ultrasound system as described above. However, the three-dimensional image might also be acquired via a different modality, for example a computer tomographic device or a magnetic resonance tomographic device.

Then, in a step 86, a shear wave elastography measurement results is acquired via a portable ultrasound image acquisition probe configured to provide for ultrasound imaging and shear wave elastography. Before that, a step of selecting a region of interest within an ultrasound image acquired with affordable ultrasound image acquisition probe may be conducted, in which region of interest an elastography measurement is conducted.

Afterwards, a step 88 of determining a location of a shear wave elastography measurement result within the three-dimensional image of the anatomical site is conducted.

Step 88 may either be conducted in that the step of determining is conducted by registering the ultrasound image with the three-dimensional image of the anatomical site. This may be conducted purely in a computer-implemented basis. Alternatively or as a redundant measure, a position of the ultrasound image acquisition probe may be tracked via a tracking device so that this enables associating the elastography measurement results with the three-dimensional image and the locations in the three-dimensional image of the anatomical site.

Before a last step 90 of displaying the location of the shear wave elastography measurement results that a user is conducting, it may further be conducted a tracking of a location of a biopsy device within the three-dimensional image of the anatomical site via the tracking device and the step of determining a distance between the location of a shear wave elastography measurement result and the location of the biopsy device. This distance may be displayed to the user in step 90 as well. Of course, in case more than one shear wave elastography measurement has taken place and, hence, there are more than one location 94, 95, 96, more than one distance of the biopsy device to each of the locations may be determined.

The method then ends in step 92.

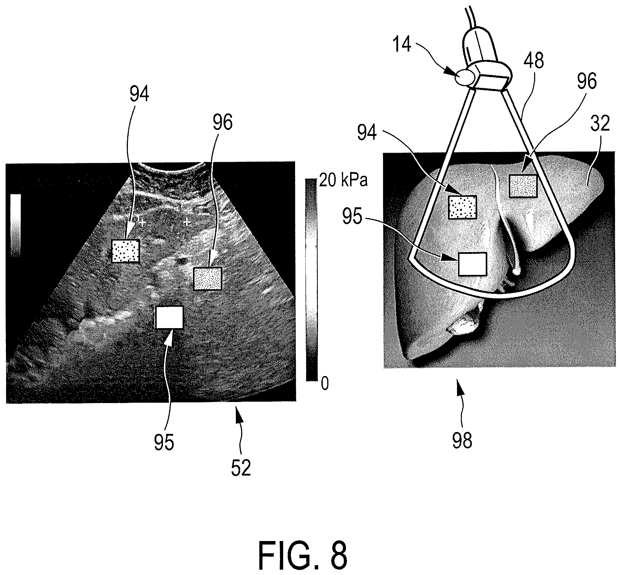

Fig. 8 shows an example of such display to the user. In the left, an ultrasound image 52, which is in the current example a commonly known bi-mode two-dimensional image, is shown. Elastography measurement results 94 to 96 are each determined and the location tracked. By this, a position of the probe 14 relative to the anatomical site 32 and its three-dimensional image 98 is known. Hence, not only a position of the plane 48 in which the elastography measurement has been conducted can be visualized within the three-dimensional image 98, but also the locations 94 to 96 can be visualized.

The mapping of elastography measurement locations to liver anatomy can be achieved by integrating live elastography related two-dimensional/three-dimensional ultrasound imaging to pre-procedure liver volume using a variety of different tracking techniques. In one embodiment, the tracking could be electromagnetic tracking wherein the probe and patient are tracked live in an electromagnetic field. The pre-procedures images could be a tracked three-dimensional to reconstruction of ultrasound images to a volume or could be a prior MR/CT images. These pre-procedure images can be registered to the electromagnetic tracking system using fiducials. Using this integration, a variety of different visualization techniques can be enabled.

In the embodiment is shown in Fig 8, the probe 14 is tracked in three dimensions, the exact location of the two-dimensional imaging plane on which elastography is performed can be determined and visualized in three-dimensional (as shown on the right). This visualization can be done for one or more imaging slices, to understand the three-dimensional context of the elastography measurement.

In another embodiment, the mapping of live elastography related two-dimensional/three-dimensional ultrasound imaging to pre-procedure images is achieved by purely image registration based tracking approach. The pre-procedures images could be a prior MR/CT volume or three-dimensional US liver anatomy acquired by stitching and compounding subsets of three-dimensional volumes using one-dimensional or two-dimensional matrix transducer sweeping. Image registration can be based on matching fiducial points, vessel structures, contour or intensity. This approach is simpler than the electromagnetic tracking based approach because it does not require additional hardware equipment, therefore it can optimize the workflow.

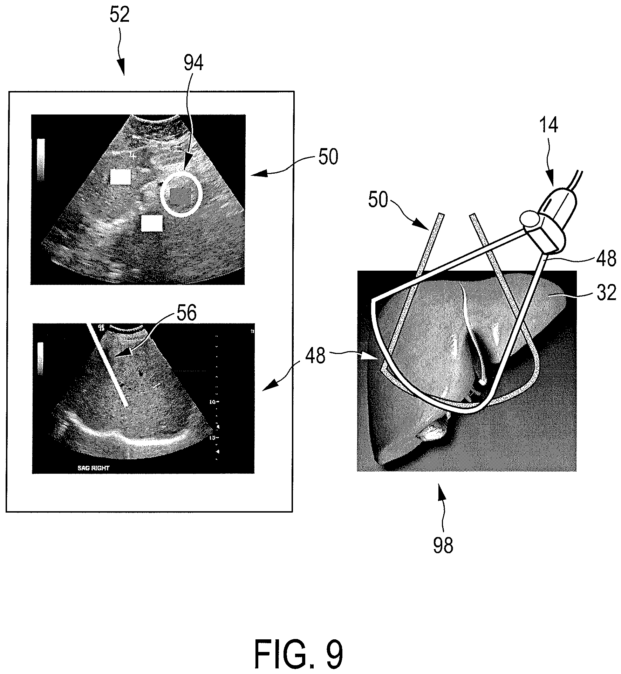

Fig. 9 shows a further example of a display to the user. On the left, again, ultrasound images 52 are displayed. They visualize not only the locations 94 of the ultrasound measurement results. But also, it can be used to follow a biopsy device 56 during examination. By this, in the three-dimensional image 98, not only the plane 50 in which the elastography measurement took place but also the plane 48 which is now used to guide the biopsy device 56 can be visualized in the three-dimensional image 98. This can ensure that a biopsy sample is within a region of interest in the line for example the location 94 of an elastography measurement. Hence, it may further be provided for that a distance of the biopsy device to this location 94 is displayed to the user.

In particular, this embodiment can be used for real time guidance of follow up biopsies. In this clinical workflow, suppose the elastography measurement 94 has been selected for biopsy (see top left image in Fig. 9). This tracked integrated system allows the user to aim the imaging plane 48 in which this particular elastography measurement was performed, and show the three-dimensional relationship of the current "biopsy" imaging plane to the elastography imaging plane.

In addition, if the biopsy is performed using a tracked device, the real time distance of the current device position to the target location (i.e. the location where the elastography measurement was made) can be displayed.

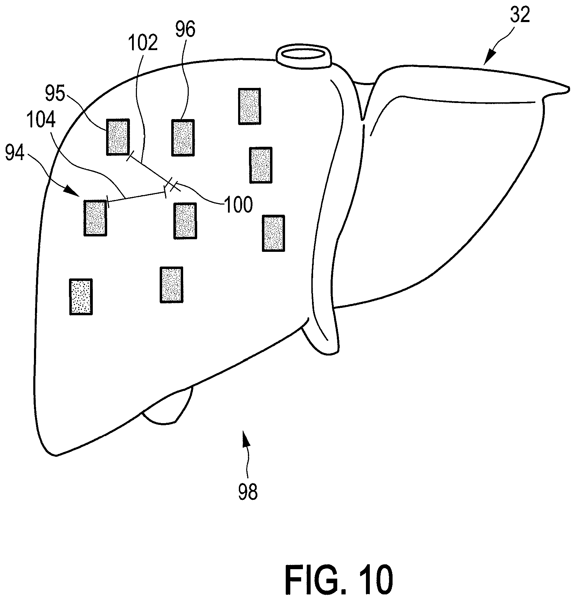

Fig. 10 shows a further example of a display to the user. As a follow-up to a biopsy examination, within the three-dimensional image 98 or the elastography measurement locations 94 to 96 may be shown in the anatomical site 32. Further, the site of the biopsy sample 100 is shown. Hence, it is possible to determine a distance to each of the locations 94 to 96 . For example, a distance 102 to the location 95 and a distance 104 between the location of the biopsy sample 100 and the elastography measurement location 94.

The three-dimensional visualization of the elastography stiffness measurement locations (white ROI boxes whose color represents stiffness value) and the biopsy location (black X sign, biopsy score can be added once it becomes available) can be in different planes 48, 50 in the three-dimensional volume. The background three-dimensional volume can be three-dimensional Ultrasound, MR or CT image.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. An ultrasound elastography system (10) for providing a shear wave elastography measurement result of an anatomical site (32), the system (10) comprising:
a portable ultrasound image acquisition probe (14) configured to provide for ultrasound imaging and shear wave elastography,
an ultrasound signal and image processing assembly (16) configured to control the ultrasound image acquisition probe (14), to determine a shear wave elastography measurement result via the portable ultrasound image acquisition probe (14) and to provide an ultrasound image (52) comprising a two-dimensional image plane (48) of the anatomical site (32),
a display (18) configured to display the ultrasound image (52) provided by the ultrasound signal and image processing assembly (16),
a memory unit (35) configured to store a second image of the anatomical site (32), and
a user input device (20) enabling a user to select a region of interest (33) within the ultrasound image (52), in which region of interest (33) an elastography measurement is to be conducted;
**characterized in that** the second image is a three-dimensional image (98) of the anatomical site (32), and **in that** the ultrasound signal and image processing assembly (16) is further configured to determine a location of the two-dimensional image plane (48) and a location (94, 95, 96) of the shear wave elastography measurement result within the three-dimensional image (98) of the anatomical site (32) and to display the location of the two-dimensional image plane (48) and the location (94, 95, 96) of the shear wave elastography measurement result within the three-dimensional image (98) of the anatomical site (32)..

2. The system according to claim 1, wherein the system (10) further comprises a tracking device (25, 25') to track a position and an orientation of the portable ultrasound image acquisition probe (14).

3. The system according to claim 2, wherein the ultrasound signal and image processing assembly (16) is further configured to determine the location of the shear wave elastography measurement result within the three-dimensional image (98) of the anatomical site (32) by registering the ultrasound image (52) with the three-dimensional image (98) of the anatomical site (32) and/or by tracking the position and then orientation of portable ultrasound image acquisition probe (14) via the tracking device (25, 25').

4. The system according to claim 2, wherein the system further comprises a biopsy device (56), and wherein the ultrasound signal and image processing assembly (16) is further configured to tracking a location (100) of a biopsy device (56) within the three-dimensional image (98) of the anatomical site (32) via the tracking device (25, 25').

5. The system according to claim 4, wherein the ultrasound signal and image processing assembly (16) is further configured to determine a distance (102) between a location (94, 95, 96) of a shear wave elastography measurement result and the location (100) of a biopsy device (56).

6. The system according to claim 1, wherein the ultrasound signal and image processing assembly (16) is further configured to determine the location of the shear wave elastography measurement result within the three-dimensional image (98) of the anatomical site (32) by registering the ultrasound image in which the region of interest (33) is selected with the three-dimensional image (98) of the anatomical site (32).

7. The system according to claim 1, wherein the ultrasound image (52) is a two-dimensional ultrasound image (52).

8. The system according to claim 2, wherein the three-dimensional image (98) of the anatomical site (32) is a three-dimensional image (98) acquired via a modality different from ultrasound image acquisition, wherein the three-dimensional image (98) of the anatomical site (32) is stored in the memory unit (35) of the ultrasound signal and image processing assembly (16).

9. The system according to claim 7, wherein the portable ultrasound image acquisition probe (14) and the ultrasound signal and image processing assembly (16) are further configured to enable a user to acquire the three-dimensional ultrasound image (52) of the anatomical site (32) and to store it as the three-dimensional image (98) of the anatomical site (32).

10. An ultrasound elastography method for inspecting an anatomical site (32) with an ultrasound elastography system (10), the method comprising the following steps:
- acquiring an ultrasound image (52) comprising a two-dimensional image plane (48) of the anatomical site (32) via a portable ultrasound image acquisition probe (14);
- acquiring (84) a second image of the anatomical site (32),
- selecting a region of interest (33) within the ultrasound image (52), in which region of interest (33) an elastography measurement is to be conducted; and
- acquiring (86) a shear wave elastography measurement result via the portable ultrasound image acquisition probe (14),
**characterized by**:
- determining (88) a location of the two-dimensional image plane (48) and a location (94, 95, 96) of a shear wave elastography measurement result within the second image, which is a three-dimensional image (98) of the anatomical site (32), and
- displaying (90) the location of the two-dimensional image plane (48) and the location of the shear wave elastography measurement result within the three-dimensional image (98) of the anatomical site (32)..

11. The method of claim 10, wherein the method further comprises the step of tracking a position and an orientation of portable ultrasound image acquisition probe (14) via a tracking device (25, 25') and determining the location of the shear wave elastography measurement result within the three-dimensional image (98) of the anatomical site (32) by registering the ultrasound image (52) with the three-dimensional image (98) of the anatomical site (32) and/or by tracking the position and the orientation of portable ultrasound image acquisition probe (14) via the tracking device (25, 25').

12. The method of claim 11, wherein the method further comprises the steps of:
- tracking a location (100) of a biopsy device (56) within the three-dimensional image (98) of the anatomical site (32) via the tracking device (25, 25'), and
- determining a distance (102) between a location (94, 95, 96) of a shear wave elastography measurement result and the location (100) of a biopsy device (56).

13. Computer program comprising program code means for causing a computer to carry out the steps of the method as claimed in any of claims 10 to 12 when said computer program is carried out on a computer.

## Patentansprüche

1. Ultraschallelastografiesystem (10) zum Bereitstellen eines Scherwellenelastografiemessergebnisses eines anatomischen Zielbereichs (32), wobei das System (10) Folgendes umfasst:
eine tragbare Ultraschallbilderfassungssonde (14), die konfiguriert ist, um Ultraschallbildgebung und Scherwellenelastografie bereitzustellen,
eine Ultraschallsignal- und Bildverarbeitungsanordnung (16), die konfiguriert ist, um die Ultraschallbilderfassungssonde (14) zu steuern, um ein Scherwellenelastografiemessergebnis über die tragbare Ultraschallbilderfassungssonde (14) zu ermitteln und um ein Ultraschallbild (52) umfassend eine zweidimensionale Bildebene (48) des anatomischen Zielbereichs (32) bereitzustellen,
eine Anzeige (18), die konfiguriert ist, um das durch die Ultraschallsignal- und Bildverarbeitungsanordnung (16) bereitgestellte Ultraschallbild (52) anzuzeigen,
eine Speichereinheit (35), die konfiguriert ist, um ein zweites Bild des anatomischen Zielbereichs (32) zu speichern, und
eine Benutzereingabevorrichtung (20), die es einem Benutzer ermöglicht, eine interessierende Region (33) in dem Ultraschallbild (52) auszuwählen, in welcher Region (33) eine Elastografiemessung durchzuführen ist;
**dadurch gekennzeichnet, dass** das zweite Bild ein dreidimensionales Bild (98) des anatomischen Zielbereichs (32) ist und dass die Ultraschallsignal- und Bildverarbeitungsanordnung (16) ferner konfiguriert ist, um einen Ort der zweidimensionalen Bildebene (48) und einen Ort (94, 95, 96) des Scherwellenelastografiemessergebnisses in dem dreidimensionalen Bild (98) des anatomischen Zielbereichs (32) zu bestimmen und um den Ort der zweidimensionalen Bildebene (48) und den Ort (94, 95, 96) des Scherwellenelastografiemessergebnisses in dem dreidimensionalen Bild (98) des anatomischen Zielbereichs (32) anzuzeigen.

2. System nach Anspruch 1, wobei das System (10) ferner eine Verfolgungsvorrichtung (25, 25') umfasst, um eine Position und eine Ausrichtung der tragbaren Ultraschallbilderfassungssonde (14) zu verfolgen.

3. System nach Anspruch 2, wobei die Ultraschallsignal- und Bildverarbeitungsanordnung (16) ferner konfiguriert ist, um den Ort des Scherwellenelastografiemessergebnisses in dem dreidimensionalen Bild (98) des anatomischen Zielbereichs (32) zu bestimmen, indem das Ultraschallbild (52) mit dem dreidimensionalen Bild (98) des anatomischen Zielbereichs (32) registriert wird und/oder indem die Position und dann die Ausrichtung der tragbaren Ultraschallbilderfassungssonde (14) über die Verfolgungsvorrichtung (25, 25') verfolgt werden.

4. System nach Anspruch 2, wobei das System ferner eine Biopsievorrichtung (56) umfasst, und wobei die Ultraschallsignal- und Bildverarbeitungsanordnung (16) ferner konfiguriert ist, um einen Ort (100) einer Biopsievorrichtung (56) in dem dreidimensionalen Bild (98) des anatomische Zielbereichs (32) über die Verfolgungsvorrichtung (25, 25') zu verfolgen.

5. System nach Anspruch 4, wobei die Ultraschallsignal- und Bildverarbeitungsanordnung (16) ferner konfiguriert ist, um einen Abstand (102) zwischen einem Ort (94, 95, 96) eines Scherwellenelastografiemessergebnisses und dem Ort (100) einer Biopsievorrichtung zu bestimmen.

6. System nach Anspruch 1, wobei die Ultraschallsignal- und Bildverarbeitungsanordnung (16) ferner konfiguriert ist, um den Ort des Scherwellenelastografiemessergebnisses in dem dreidimensionalen Bild (98) des anatomischen Zielbereichs (32) zu bestimmen, indem das Ultraschallbild, in dem die interessierende Region (33) ausgewählt wurde, mit dem dreidimensionalen Bild (98) des anatomischen Zielbereichs (32) registriert wird.

7. System nach Anspruch 1, wobei das Ultraschallbild (52) ein zweidimensionales Ultraschallbild (52) ist.

8. System nach Anspruch 2, wobei das dreidimensionale Bild (98) des anatomischen Zielbereichs (32) ein dreidimensionales Bild (98) ist, das über eine andere Modalität als Ultraschallbilderfassung erfasst wurde, wobei das dreidimensionale Bild (98) des anatomischen Zielbereichs (32) in der Speichereinheit (35) der Ultraschallsignal- und Bildverarbeitungsanordnung (16) gespeichert wird.

9. System nach Anspruch 7, wobei die tragbare Ultraschallbilderfassungssonde (14) und die Ultraschallsignal- und Bildverarbeitungsanordnung (16) ferner konfiguriert sind, um einem Benutzer zu ermöglichen, das dreidimensionale Ultraschallbild (52) des anatomischen Zielbereichs (32) zu erfassen und es als das dreidimensionale Bild (98) des anatomischen Zielbereichs (32) zu speichern.

10. Ultraschallelastografieverfahren zur Untersuchung eines anatomischen Zielbereichs (32) mit einem Ultraschallelastografiesystem (10), wobei das Verfahren die folgenden Schritte umfasst:
- das Erfassen eines Ultraschallbilds (52) umfassend eine zweidimensionale Bildebene (48) des anatomischen Zielbereichs (32) über eine tragbare Ultraschallbilderfassungssonde (14),
- das Erfassen (84) eines zweiten Bilds des anatomischen Zielbereichs (32),
- das Auswählen einer interessierenden Region (33) in dem Ultraschallbild (52), in welcher Region (33) eine Elastografiemessung durchzuführen ist, und
- das Erfassen (86) eines Scherwellenelastografiemessergebnisses über die tragbare Ultraschallbilderfassungssonde (14),
**gekennzeichnet durch**:
- das Bestimmen (88) eines Orts der zweidimensionalen Bildebene (48) und eines Orts (94, 95, 96) eines Scherwellenelastografiemessergebnisses in dem zweiten Bild, welches ein dreidimensionales Bild (98) des anatomischen Zielbereichs (32) ist, und
- das Anzeigen (90) des Orts der zweidimensionalen Bildebene (48) und des Ort (94, 95, 96) des Scherwellenelastografiemessergebnisses in dem dreidimensionalen Bild (98) des anatomischen Zielbereichs (32).

11. Verfahren nach Anspruch 10, wobei das Verfahren ferner den Schritt des Verfolgens einer Position und einer Ausrichtung der tragbaren Ultraschallbilderfassungssonde (14) über eine Verfolgungsvorrichtung (25, 25') und das Bestimmen des Orts des Scherwellenelastografiemessergebnisses in dem dreidimensionalen Bild (98) des anatomischen Zielbereichs (32) umfasst, indem das Ultraschallbild (52) mit dem dreidimensionalen Bild (98) des anatomischen Zielbereichs (32) registriert wird und/oder indem die Position und dann die Ausrichtung der tragbaren Ultraschallbilderfassungssonde (14) über die Verfolgungsvorrichtung (25, 25') verfolgt werden.

12. Verfahren nach Anspruch 11, wobei das Verfahren ferner die folgenden Schritte umfasst:
- Verfolgen eines Orts (100) einer Biopsievorrichtung (56) in dem dreidimensionalen Bild (98) des anatomischen Zielbereichs (32) über die Verfolgungsvorrichtung (25, 25'), und
- Bestimmen eines Abstands (102) zwischen einem Ort (94, 95, 96) eines Scherwellenelastografiemessergebnisses und dem Ort (100) einer Biopsievorrichtung (56).

13. Computerprogramm umfassend Programmcodemittel zum Veranlassen eines Computers, die Schritte des Verfahrens nach einem der Ansprüche 10 bis 12 durchzuführen, wenn das genannte Computerprogramm auf einem Computer ausgeführt wird.

## Revendications

1. Système d'élastographie par ultrasons (10) pour fournir un résultat de mesure d'élastographie par onde de cisaillement d'un site anatomique (32), le système (10) comprenant :
une sonde portative d'acquisition d'image par ultrasons (14) configurée pour permettre une imagerie par ultrasons et une élastographie par onde de cisaillement,
un ensemble de traitement d'image et de signal à ultrasons (16) configuré pour commander la sonde d'acquisition d'image par ultrasons (14), pour déterminer un résultat de mesure d'élastographie par onde de cisaillement via la sonde portative d'acquisition d'image par ultrasons (14) et pour fournir une image par ultrasons (52) comprenant un plan d'image bidimensionnel (48) du site anatomique (32),
un afficheur (18) configuré pour afficher l'image par ultrasons (52) fournie par l'ensemble de traitement d'image et de signal par ultrasons (16),
une unité de mémoire (35) configurée pour stocker une seconde image du site anatomique (32), et
un dispositif d'entrée utilisateur (20) permettant à un utilisateur de sélectionner une région d'intérêt (33) à l'intérieur de l'image par ultrasons (52), région d'intérêt (33) dans laquelle une mesure d'élastographie doit être menée ;
**caractérisé en ce que** la seconde image est une image tridimensionnelle (98) du site anatomique (32) et **en ce que** l'ensemble de traitement d'image et de signal par ultrasons (16) est en outre configuré pour déterminer un emplacement du plan d'image bidimensionnel (48) et un emplacement (94, 95, 96) du résultat de mesure d'élastographie par onde de cisaillement à l'intérieur de l'image tridimensionnelle (98) du site anatomique (32) et pour afficher l'emplacement du plan d'image bidimensionnel (48) et l'emplacement (94, 95, 96) du résultat de mesure d'élastographie par onde de cisaillement à l'intérieur de l'image tridimensionnelle (98) du site anatomique (32).

2. Système selon la revendication 1, dans lequel le système (10) comprend en outre un dispositif de suivi (25, 25') pour suivre une position et une orientation de la sonde portative d'acquisition d'image par ultrasons (14).

3. Système selon la revendication 2, dans lequel l'ensemble de traitement d'image et de signal par ultrasons (16) est en outre configuré pour déterminer l'emplacement du résultat de mesure d'élastographie par onde de cisaillement à l'intérieur de l'image tridimensionnelle (98) du site anatomique (32) en mettant en correspondance l'image par ultrasons (52) et l'image tridimensionnelle (98) du site anatomique (32) et/ou en suivant la position et ensuite l'orientation de la sonde portative d'acquisition d'image par ultrasons (14) via le dispositif de suivi (25, 25').

4. Système selon la revendication 2, dans lequel le système comprend en outre un dispositif de biopsie (56), et dans lequel l'ensemble de traitement d'image et de signal par ultrasons (16) est en outre configuré pour suivre un emplacement (100) d'un dispositif de biopsie (56) à l'intérieur de l'image tridimensionnelle (98) du site anatomique (32) via le dispositif de suivi (25, 25').

5. Système selon la revendication 4, dans lequel l'ensemble de traitement d'image et de signal par ultrasons (16) est en outre configuré pour déterminer une distance (102) entre un emplacement (94, 95, 96) d'un résultat de mesure d'élastographie par onde de cisaillement et l'emplacement (100) d'un dispositif de biopsie (56).

6. Système selon la revendication 1, dans lequel l'ensemble de traitement d'image et de signal par ultrasons (16) est en outre configuré pour déterminer l'emplacement du résultat de mesure d'élastographie par onde de cisaillement à l'intérieur de l'image tridimensionnelle (98) du site anatomique (32) en mettant en correspondance l'image par ultrasons dans laquelle la région d'intérêt (33) est sélectionnée et l'image tridimensionnelle (98) du site anatomique (32).

7. Système selon la revendication 1, dans lequel l'image par ultrasons (52) est une image par ultrasons bidimensionnelle (52).

8. Système selon la revendication 2, dans lequel l'image tridimensionnelle (98) du site anatomique (32) est une image tridimensionnelle (98) acquise via une modalité différente de l'acquisition d'image par ultrasons, dans laquelle l'image tridimensionnelle (98) du site anatomique (32) est stockée dans l'unité de mémoire (35) de l'ensemble de traitement d'image et de signal par ultrasons (16).

9. Système selon la revendication 7, dans lequel la sonde portative d'acquisition d'image par ultrasons (14) et l'ensemble de traitement d'image et de signal par ultrasons (16) sont en outre configurés pour permettre à un utilisateur d'acquérir l'image par ultrasons tridimensionnelle (52) du site anatomique (32) et de le stocker en tant qu'image tridimensionnelle (98) du site anatomique (32).

10. Procédé d'élastographie par ultrasons pour examiner un site anatomique (32) avec un système d'élastographie par ultrasons (10), le procédé comprenant les étapes suivantes :
- acquisition d'une image par ultrasons (52) comprenant un plan d'image bidimensionnel (48) du site anatomique (32) via une sonde portative d'acquisition d'image par ultrasons (14) ;
- acquisition (84) d'une seconde image du site anatomique (32),
- sélection d'une région d'intérêt (33) à l'intérieur de l'image par ultrasons (52), région d'intérêt (33) dans laquelle une mesure d'élastographie doit être menée ; et
- acquisition (86) d'un résultat de mesure d'élastographie par onde de cisaillement via la sonde portative d'acquisition d'image par ultrasons (14),
**caractérisé par** :
- la détermination (88) d'un emplacement du plan d'image bidimensionnel (48) et d'un emplacement (94, 95, 96) d'un résultat de mesure d'élastographie par onde de cisaillement dans la seconde image, qui est une image tridimensionnelle (98) du site anatomique (32), et
- l'affichage (90) de l'emplacement du plan d'image bidimensionnel (48) et de l'emplacement du résultat de mesure d'élastographie par onde de cisaillement à l'intérieur de l'image tridimensionnelle (98) du site anatomique (32).

11. Procédé selon la revendication 10, dans lequel le procédé comprend en outre l'étape de suivi d'une position et d'une orientation de sonde portative d'acquisition d'image par ultrasons (14) via un dispositif de suivi (25, 25') et de détermination de l'emplacement du résultat de mesure d'élastographie par onde de cisaillement dans l'image tridimensionnelle (98) du site anatomique (32) par mise en correspondance de l'image par ultrasons (52) et de l'image tridimensionnelle (98) du site anatomique (32) et/ou en suivant la position et l'orientation de la sonde portative d'acquisition d'image par ultrasons (14) via le dispositif de suivi (25, 25').

12. Procédé selon la revendication 11, dans lequel le procédé comprend en outre les étapes de :
- suivi d'un emplacement (100) d'un dispositif de biopsie (56) à l'intérieur de l'image tridimensionnelle (98) du site anatomique (32) via le dispositif de suivi (25, 25'), et
- détermination d'une distance (102) entre un emplacement (94, 95, 96) d'un résultat de mesure d'élastographie par onde de cisaillement et l'emplacement (100) d'un dispositif de biopsie (56).

13. Programme informatique comprenant un moyen formant code de programme pour amener un ordinateur à effectuer les étapes du procédé selon l'une quelconque des revendications 10 à 12 quand ledit programme informatique est exécuté sur un ordinateur.
